# EUROPEAN PATENT APPLICATION

(11) **EP 4 659 775 A1**
(43) Date of publication of application: **10.12.2025**
(21) Application number: 24753230.2
(22) Date of filing: 01.02.2024
(51) Int. Cl.: A61M 5/142

(54) **TUBE VOID DETERMINATION DEVICE AND LIQUID DELIVERY PUMP**

(30) Priority: 06.02.2023 JP 2023016418
(71) Applicant: Terumo Kabushiki Kaisha, Tokyo 151-0072 (JP)
(72) Inventor: KUNIMATSU Yuki, Ashigarakami-gun, Kanagawa 259-0151 (JP); WADA Yuya, Ashigarakami-gun, Kanagawa 259-0151 (JP); NAKAMURA Takuro, Ashigarakami-gun, Kanagawa 259-0151 (JP)
(74) Representative: KIPA AB
(86) International application number: PCT/JP2024/003323
(87) International publication number: WO 2024/166787

(57) **Abstract**

A tube void determination device according to the present disclosure includes: a main body including an accommodating groove capable of accommodating a tube; a door attached to the main body to be openable and closable, the door being capable of pressing the tube accommodated in the accommodating groove toward a groove bottom of the accommodating groove in conjunction with a closing operation with respect to the main body; a transmitter capable of transmitting an electromagnetic wave or a sound wave to the tube accommodated in the accommodating groove, and a receiver capable of receiving the electromagnetic wave or the sound wave transmitted from the transmitter and transmitted through or reflected by the tube; and a controller that determines the presence or absence of a void in the tube based on a reception intensity received by the receiver in a process of the closing operation of the door with respect to the main body.

## Description

### Technical Field

The present disclosure relates to a tube void determination device and a liquid delivery pump.

### Background Art

Conventionally, a tube void determination device capable of determining the presence or absence of voids such as air bubbles in tubes using ultrasonic waves or the like has been known. Patent Literature 1 discloses an air bubble amount detection system as this type of tube void determination device.

### Citation List

### Patent Literature

Patent Literature 1: JP 2005-084017 A

### Summary of Invention

### Technical Problem

There is a case where a tube is attached to the tube void determination device in a state in which liquid such as a medicine adheres to an outer surface of the tube. When a portion of the tube in which the liquid adheres to the outer surface is attached at a position of the tube void determination device, there is a problem that some of transmission waves from a transmitter such as an ultrasonic transmitter do not pass through the inside of the tube, but propagate in the adhering liquid to reach a receiver such as an ultrasonic receiver. When such wrap-around of the transmission waves occurs, there is a possibility that the tube void determination device cannot accurately determine the presence or absence of a void in the tube even in a case where the void exists in the tube, for example, in a case where a tube (hereinafter, referred to as an "empty tube") which is not filled with liquid is attached.

An object of the present invention is to provide a tube void determination device and a liquid delivery pump which are capable of determining an empty tube even in a state in which liquid adheres to an outer surface of the empty tube.

### Solution to Problem

A tube void determination device according to a first aspect of the present disclosure is
(1)
   a tube void determination device including:
   a main body including an accommodating groove capable of accommodating a tube;
   a door attached to the main body to be openable and closable, the door being capable of pressing the tube accommodated in the accommodating groove toward a groove bottom of the accommodating groove in conjunction with a closing operation with respect to the main body;
   a transmitter capable of transmitting an electromagnetic wave or a sound wave to the tube accommodated in the accommodating groove, and a receiver capable of receiving the electromagnetic wave or the sound wave transmitted from the transmitter and transmitted through or reflected by the tube; and
   a controller that determines the presence or absence of a void in the tube based on a reception intensity received by the receiver in a process of the closing operation of the door with respect to the main body.

A tube void determination device according to an embodiment of the present disclosure is
(2)
the tube void determination device according to (1), in which the transmitter and the receiver are arranged to face each other in a radial direction of the tube while sandwiching the tube accommodated in the accommodating groove.

A tube void determination device according to an embodiment of the present disclosure is
(3)
the tube void determination device according to (2), in which
the main body is capable of clamping the tube between two opposing groove walls of the accommodating groove,
the transmitter is capable of transmitting the electromagnetic wave or the sound wave to the tube through one groove wall of the two groove walls, and
the receiver is capable of receiving the electromagnetic wave or the sound wave transmitted through or reflected by the tube through the other groove wall of the two groove walls.

A tube void determination device according to an embodiment of the present disclosure is
(4)
the tube void determination device according to any one of (1) to (3), further including an opening/closing degree detection sensor capable of detecting the degree of opening/closing of the door with respect to the main body,
in which the controller determines the presence or absence of the void in the tube based on a variation and a maximum value of the reception intensity received by the receiver in the process of the closing operation of the door with respect to the main body, and the degree of opening/closing of the door with respect to the main body.

A liquid delivery pump as a second aspect of the present disclosure is
(5)
a liquid delivery pump including:
the tube void determination device according to any one of (1) to (4); and
a liquid delivery unit capable of delivering liquid inside the tube.

### Advantageous Effects of Invention

According to the present invention, it is possible to provide the tube void determination device and the liquid delivery pump which are capable of determining the empty tube even in the state in which the liquid adheres to the outer surface of the empty tube.

### Brief Description of Drawings

Fig. 1 is a view illustrating a tube void determination device as an embodiment of the present disclosure, and is a view illustrating an open state in which a door is opened with respect to a main body.
Fig. 2 is a view illustrating a closed state in which the door is closed with respect to the main body in the tube void determination device illustrated in Fig. 1.
Fig. 3 is a view illustrating a state in which a liquid-filled tube without external liquid adhesion is accommodated in an accommodating groove of the tube void determination device in the open state illustrated in Fig. 1.
Fig. 4 is a view illustrating a state in which the liquid-filled tube without external liquid adhesion is accommodated in the accommodating groove of the tube void determination device in the closed state illustrated in Fig. 2.
Fig. 5 is a view illustrating a state in which an empty tube with external liquid adhesion is accommodated in the accommodating groove of the tube void determination device in the open state illustrated in Fig. 1.
Fig. 6 is a view illustrating a state in which the empty tube with external liquid adhesion is accommodated in the accommodating groove of the tube void determination device in the closed state illustrated in Fig. 2.
Fig. 7 is a view illustrating an example of variations in reception intensity received by a receiver in a process of a closing operation of the door with respect to the main body.
Fig. 8 is a view illustrating an example of a void determination method by a controller of the tube void determination device.
Fig. 9 is a view illustrating a liquid delivery line to which a liquid delivery pump as an embodiment of the present disclosure including the tube void determination device illustrated in Fig. 1 is attached.
Fig. 10 is a front side perspective view of the liquid delivery pump illustrated in Fig. 9.
Fig. 11 is a front view of the liquid delivery pump illustrated in Fig. 9, and is a view illustrating a closed state in which a door is closed with respect to a main body.
Fig. 12 is a front view of the liquid delivery pump illustrated in Fig. 9, and is a view illustrating an open state in which the door is opened with respect to the main body.

### Description of Embodiments

Hereinafter, embodiments of a tube void determination device and a liquid delivery pump according to the present disclosure will be described by way of example with reference to the drawings. In the drawings, the same components are denoted by the same reference signs.

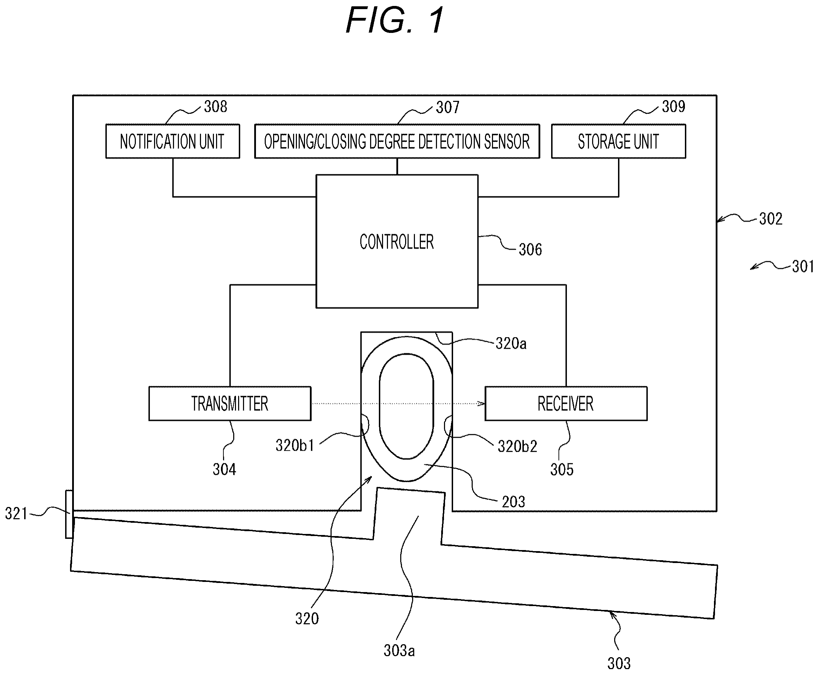

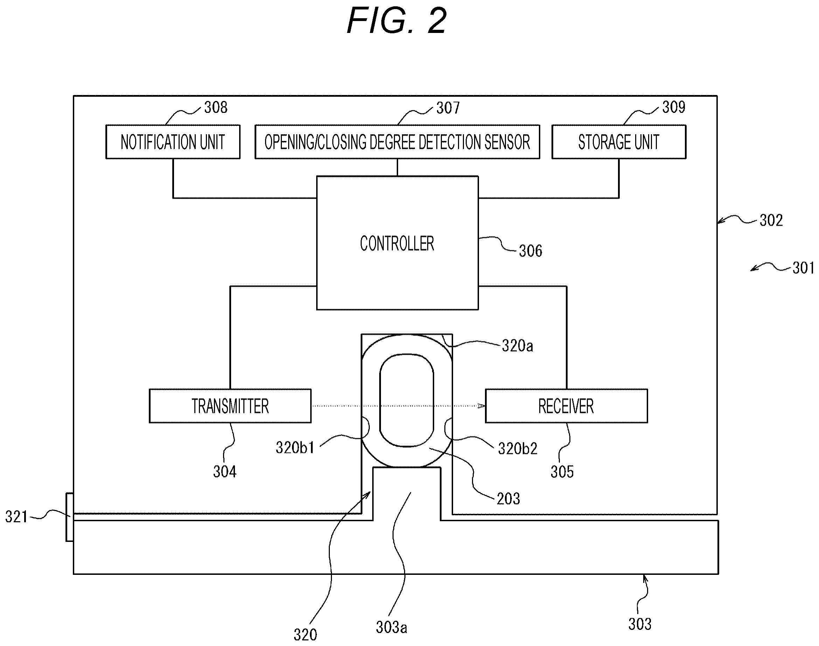

Figs. 1 and 2 are views illustrating a tube void determination device 301 as an embodiment of the tube void determination device according to the present disclosure. As illustrated in Figs. 1 and 2, the tube void determination device 301 includes a main body 302, a door 303, a transmitter 304 and a receiver 305, and a controller 306.

The main body 302 includes an accommodating groove 320 capable of accommodating a tube 203. The door 303 is attached to the main body 302 to be openable and closable. Fig. 1 illustrates a state in which the door 303 is opened with respect to the main body 302. Fig. 2 illustrates a state in which the door 303 is closed with respect to the main body 302. In addition, the door 303 is configured to be capable of pressing the tube 203 accommodated in the accommodating groove 320 of the main body 302 toward a groove bottom 320a of the accommodating groove 320 in conjunction with a closing operation with respect to the main body 302.

The door 303 of the present embodiment is attached to the main body 302 via a hinge 321, but an attachment configuration that can be opened and closed with respect to the main body 302 is not particularly limited. In addition, the door 303 of the present embodiment includes a protruding portion 303a. Then, the protruding portion 303a is configured to press the tube 203 in the closing operation of the door 303 with respect to the main body 302. However, it is sufficient to adopt a configuration in which the tube 203 is pressed by the door 303 in the closing operation of the door 303, and a configuration of a portion of the door 303 that presses the tube 203 is not limited to the protruding portion 303a of the present embodiment.

The transmitter 304 is configured to transmit an electromagnetic wave such as light or a sound wave such as an ultrasonic wave to the tube 203 accommodated in the accommodating groove 320. In addition, the receiver 305 is configured to receive the electromagnetic waves or the sound waves transmitted from the transmitter 304 and transmitted through or reflected by the tube 203. The transmitter 304 may be, for example, an ultrasonic transmitter capable of transmitting an ultrasonic wave. The receiver 305 may be, for example, an ultrasonic receiver capable of receiving an ultrasonic wave. Specifically, for example, the transmitter 304 and the receiver 305 may include a piezoelectric element.

However, the transmitter 304 and the receiver 305 are not limited to the ultrasonic transmitter and the ultrasonic receiver, respectively, and may be, for example, a light emitting unit and a light receiving unit. The light emitting unit may include, for example, a light emitting element that emits light, such as a light emitting diode (LED) or a laser diode (LD). The light receiving unit may include, for example, a light receiving element such as a photo transistor (PT) or a photo diode (PD). As described above, the tube void determination device 301 may be configured to use a sound wave such as an ultrasonic wave, or may be configured to use an electromagnetic wave such as light. A "reception intensity" described below means a reception intensity of an ultrasonic wave received by the ultrasonic receiver in the case of the configuration using an ultrasonic wave, and means a reception intensity of light received by the light receiving unit in the case of the configuration using light.

Further, the receiver 305 of the present embodiment is configured to be capable of receiving the electromagnetic waves or sound waves transmitted through the tube 203, but is not limited to this configuration. The receiver 305 may be configured to be capable of receiving the electromagnetic waves or the sound waves reflected by the tube 203.

The controller 306 can determine the presence or absence of a void in the tube 203 based on the reception intensity received by the receiver 305 in a process of the closing operation of the door 303 with respect to the main body 302. This will be described in detail hereinafter.

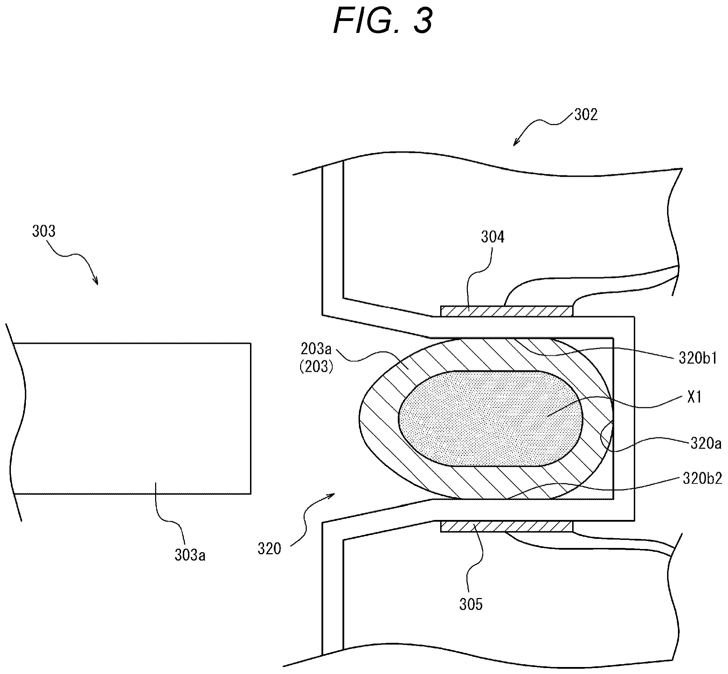

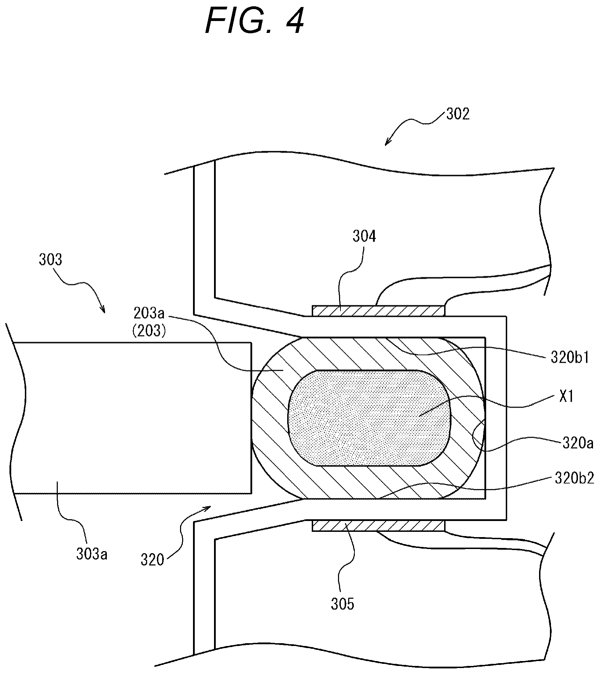

Figs. 3 and 4 are views illustrating a state in which the tube 203 (hereinafter, referred to as a "liquid-filled tube 203a without external liquid adhesion") whose inside is filled with liquid X1 without liquid adhering to the outer surface is accommodated in the accommodating groove 320. Fig. 3 illustrates a state in which the door 303 is opened with respect to the main body 302. Fig. 4 illustrates a closed state in which the door 303 is closed with respect to the main body 302. The liquid X1 inside the tube 203 is not particularly limited, and examples thereof include a medicine and a nutrient.

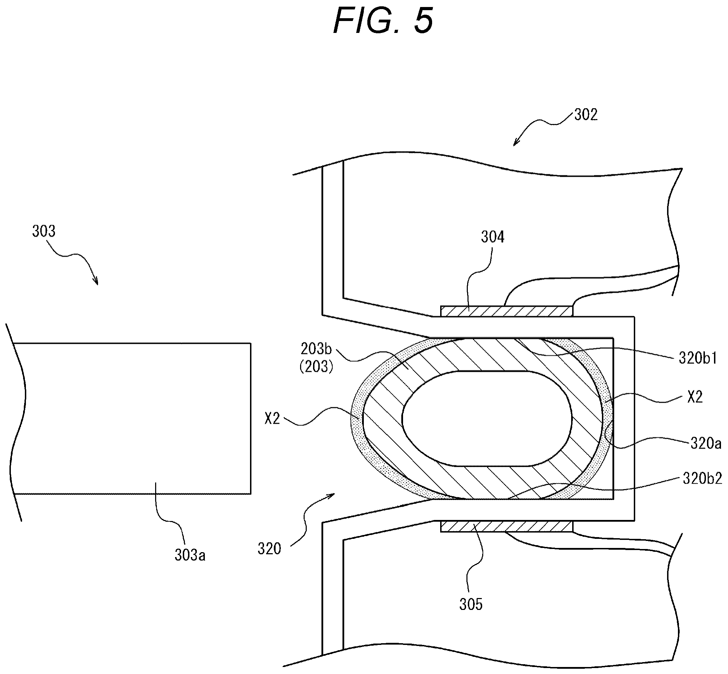

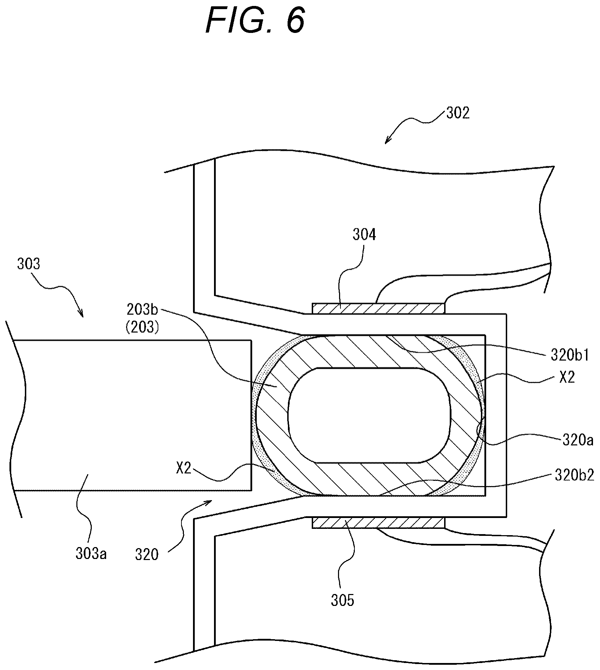

On the other hand, Figs. 5 and 6 are views illustrating a state in which the tube 203 (hereinafter, referred to as an "empty tube 203b with external liquid adhesion") in a state in which the inside is not filled with liquid and liquid X2 adheres to the outer surface is accommodated in the accommodating groove 320. Fig. 5 illustrates a state in which the door 303 is opened with respect to the main body 302. Fig. 6 illustrates a closed state in which the door 303 is closed with respect to the main body 302. The liquid X2 adhering to the outer surface of the tube 203 is not particularly limited, and examples thereof include a medicine and a nutrient.

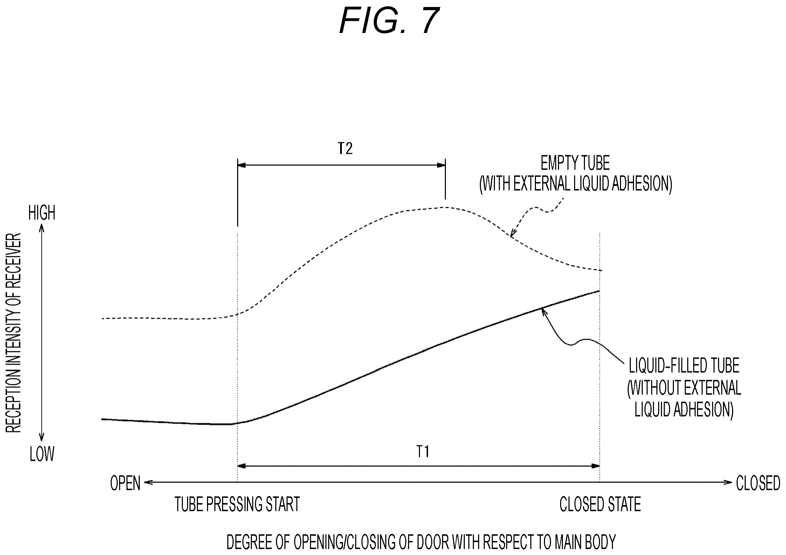

Fig. 7 is a view illustrating an example of variations in the reception intensity received by the receiver 305 in the process of the closing operation of the door 303 with respect to the main body 302. As the closing operation proceeds from a state in which the door 303 is opened with respect to the main body 302 (see Figs. 1, 3, and 5) to a state in which the door is closed with respect to the main body 302 (see Figs. 2, 4, and 6), the reception intensity received by the receiver 305 varies. Fig. 7 illustrates variations in reception intensity in the process of the closing operation for the liquid-filled tube 203a without external liquid adhesion (see "liquid-filled tube (without external liquid adhesion)" in Fig. 7). In addition, Fig. 7 illustrates variations in reception intensity in the process of the closing operation for the empty tube 203b with external liquid adhesion (see "empty tube (with external liquid adhesion)" in Fig. 7).

In the example illustrated in Fig. 7, in the closed state in which the closing operation of the door 303 with respect to the main body 302 is completed, the reception intensity received by the receiver 305 for the liquid-filled tube 203a without external liquid adhesion and the reception intensity received by the receiver 305 for the empty tube 203b with external liquid adhesion are substantially equal. This means that, in the case of the empty tube 203b with external liquid adhesion, which is the tube 203 whose inside is not filled with liquid, as illustrated in Fig. 6, an ultrasonic wave may propagate through the liquid X2 adhering to the outer surface and be received by the receiver 305 to increase the reception intensity of the receiver 305. As described above, there is a case where it is difficult to discriminate between the liquid-filled tube 203a without external liquid adhesion and the empty tube 203b with external liquid adhesion only by the comparison between the reception intensities of the receiver 305 in the closed state.

On the other hand, a variation history of the reception intensity received by the receiver 305 in the process of the closing operation is different between the liquid-filled tube 203a without external liquid adhesion and the empty tube 203b with external liquid adhesion as illustrated in Fig. 7. That is, the controller 306 can discriminate between the liquid-filled tube 203a without external liquid adhesion and the empty tube 203b with external liquid adhesion by considering the variations in the reception intensity received by the receiver 305 in the process of the closing operation.

As illustrated in Fig. 7, in the case of the liquid-filled tube 203a without external liquid adhesion (see "liquid-filled tube (without external liquid adhesion)" in Fig. 7), the liquid X2 (see Figs. 5 and 6) does not adhere to the outer surface, so that unintended propagation of the ultrasonic wave in the process of the closing operation does not occur. The tube 203 illustrated in Figs. 3 to 6 is pressed by the door 303 by the closing operation, and is deformed such that the area in close contact with two opposing groove walls 320b1 and 320b2 of the accommodating groove 320 increases. Therefore, the liquid-filled tube 203a without external liquid adhesion is pressed by the door 303 by the closing operation, and is set in a position and a posture in which the internal liquid X1 can be accurately detected by the transmitter 304 and the receiver 305 in the closed state. That is, as illustrated in Fig. 7, in the case of the liquid-filled tube 203a without external liquid adhesion, the reception intensity received by the receiver 305 gradually increases until the closed state in the process of the closing operation.

On the other hand, in the case of the empty tube 203b with external liquid adhesion (see "empty tube (with external liquid adhesion)" in Fig. 7), the liquid X2 (see Figs. 5 and 6) adheres to the outer surface, so that the unintended propagation of the ultrasonic wave in the process of the closing operation may occur. Therefore, as illustrated in Fig. 7, in the process in which the empty tube 203b with external liquid adhesion is pressed by the door 303 by the closing operation, a case where the reception intensity received by the receiver 305 increases as compared with the above-described liquid-filled tube 203a without external liquid adhesion (see "liquid-filled tube (without external liquid adhesion)" in Fig. 7) may occur. Further, in the process in which the empty tube 203b with external liquid adhesion is pressed by the door 303 by the closing operation, an adhesion position or the like of the liquid X2 adhering to the outer surface of the empty tube 203b with external liquid adhesion varies, so that a decrease in the propagation of the ultrasonic wave through the liquid X2 adhering to the outer surface of the empty tube 203b with external liquid adhesion may occur. That is, as illustrated in Fig. 7, in the case of the empty tube 203b with external liquid adhesion, a decrease in the reception intensity received by the receiver 305 may occur in the process of the closing operation.

As described above, the variation history of the reception intensity received by the receiver 305 in the process of the closing operation is different between the liquid-filled tube 203a without external liquid adhesion and the empty tube 203b with external liquid adhesion. Therefore, the controller 306 can discriminate between the liquid-filled tube 203a without external liquid adhesion and the empty tube 203b with external liquid adhesion based on the variations in the reception intensity received by the receiver 305 in the process of the closing operation.

In the case of the example illustrated in Fig. 7, the controller 306 may discriminate between the liquid-filled tube 203a without external liquid adhesion and the empty tube 203b with external liquid adhesion based on, for example, a difference (difference between "T1" and "T2" in Fig. 7) in gradually increasing time of the reception intensity received by the receiver 305 in the process of the closing operation. In addition, in the case of the example illustrated in Fig. 7, the controller 306 may discriminate between the liquid-filled tube 203a without external liquid adhesion and the empty tube 203b with external liquid adhesion based on, for example, whether or not the decrease in the reception intensity received by the receiver 305 occurs in the process of the closing operation.

However, the variation history of the reception intensity according to the degree of opening/closing of the door 303 illustrated in Fig. 7 is an example, and a variation history of the reception intensity according to the degree of opening/closing of the door 303 of each of the liquid-filled tube 203a without external liquid adhesion and the empty tube 203b with external liquid adhesion is not limited to the variation history of the reception intensity illustrated in Fig. 7. That is, variations in the reception intensities in the process of the closing operation of the liquid-filled tube 203a without external liquid adhesion and the empty tube 203b with external liquid adhesion may be different from the variations illustrated in Fig. 7. Even in such a case, there is a difference between the variations in the reception intensities in the process of the closing operation of the liquid-filled tube 203a without external liquid adhesion and the empty tube 203b with external liquid adhesion. Therefore, the controller 306 can discriminate between the liquid-filled tube 203a without external liquid adhesion and the empty tube 203b with external liquid adhesion by comparing the variations in the reception intensities in the process of the closing operation.

Further, the controller 306 can determine the tube 203 (hereinafter, referred to as a "liquid-filled tube with external liquid adhesion") whose inside is filled with the liquid X1 (see Figs. 3 and 4) with the liquid X2 (see Figs. 5 and 6) adhering to the outer surface by using a maximum value of a reception intensity in the process of the closing operation. In the case of the liquid-filled tube with external liquid adhesion, the maximum value of the reception intensity in the process of the closing operation is higher than a maximum value of the reception intensity in the process of the closing operation of each of the liquid-filled tube 203a without external liquid adhesion and the empty tube 203b with external liquid adhesion. Therefore, when a maximum value of a reception intensity in the process of the closing operation is equal to or higher than a predetermined first threshold, the controller 306 determines that the tube 203 accommodated in the accommodating groove 320 is the liquid-filled tube with external liquid adhesion.

In addition, the controller 306 can also determine the tube 203 (hereinafter, referred to as an "empty tube without external liquid adhesion") whose inside is not filled with the liquid X1 (see Figs. 3 and 4) without the liquid X2 (see Figs. 5 and 6) adhering to the outer surface by using a maximum value of a reception intensity in the process of the closing operation. In the case of the empty tube without external liquid adhesion, the reception intensity in the process of the closing operation is substantially zero, and the maximum value thereof is lower than the maximum value of the reception intensity in the process of the closing operation of each of the liquid-filled tube 203a without external liquid adhesion and the empty tube 203b with external liquid adhesion. Therefore, when a maximum value of a reception intensity in the process of the closing operation is equal to or lower than a predetermined second threshold, the controller 306 determines that the tube 203 accommodated in the accommodating groove 320 is the empty tube without external liquid adhesion. The predetermined second threshold is a value lower than the above-described predetermined first threshold.

As described above, the controller 306 can discriminate between the liquid-filled tube 203a without external liquid adhesion and the empty tube 203b with external liquid adhesion based on the variations in the reception intensity received by the receiver 305 in the process of the closing operation of the door 303 with respect to the main body 302. Further, the controller 306 can discriminate between the liquid-filled tube with external liquid adhesion and the empty tube without external liquid adhesion by using the maximum value of the reception intensity received by the receiver 305 in the process of the closing operation of the door 303 with respect to the main body 302. Therefore, according to the controller 306, the presence or absence of a void in the tube 203 can be determined regardless of whether or not the liquid X2 (see Figs. 5 and 6) adheres to the outer surface based on the reception intensity received by the receiver 305 in the process of the closing operation of the door 303 with respect to the main body 302. That is, the tube void determination device 301 can determine an empty tube regardless of whether or not the liquid X2 (see Figs. 5 and 6) adheres to the outer surface.

The controller 306 controls and manages the entire tube void determination device 301 including the respective functional units of the tube void determination device 301. The controller 306 includes at least one processor. The controller 306 may be configured by a processor such as a central processing unit (CPU) that executes a program defining a control procedure or a dedicated processor specialized for processing of each function. Such a program may be stored in, for example, a storage unit 309 described later or a storage medium outside the tube void determination device 301.

Next, further details of the tube void determination device 301 of the present embodiment will be described with reference to Figs. 1 to 7.

As illustrated in Figs. 1 to 6, in the tube void determination device 301 of the present embodiment, the transmitter 304 and the receiver 305 are arranged to face each other in a radial direction of the tube 203 while sandwiching the tube 203 accommodated in the accommodating groove 320. The transmitter 304 according to the present embodiment transmits the ultrasonic wave toward the opposing receiver 305. The receiver 305 receives the ultrasonic wave transmitted from the transmitter 304 and transmitted through the tube 203. As described above, since the transmitter 304 and the receiver 305 are arranged so as to face each other while sandwiching the tube 203 accommodated in the accommodating groove 320, it is possible to more accurately determine the presence or absence of a void in the tube 203.

In addition, as illustrated in Figs. 1 to 6, the main body 302 of the present embodiment can clamp the tube 203 between the two opposing groove walls 320b1 and 320b2 of the accommodating groove 320. The transmitter 304 can transmit the ultrasonic wave to the tube 203 through one groove wall 320b1 of the two groove walls 320b1 and 320b2. The receiver 305 can receive the ultrasonic wave, transmitted through the tube 203, through the other groove wall 320b2 of the two groove walls 320b1 and 320b2. As described above, since the transmitter 304 and the receiver 305 are covered with the groove walls 320b1 and 320b2, damage or the like of the transmitter 304 and the receiver 305 can be suppressed as compared with a case of being exposed without being covered with the groove walls 320b1 and 320b2. The two groove walls 320b1 and 320b2 are configured to transmit electromagnetic waves or sound waves to be used. Specifically, the two groove walls 320b1 and 320b2 of the present embodiment are formed using a resin capable of transmitting the ultrasonic wave.

As illustrated in Figs. 1 and 2, the tube void determination device 301 of the present embodiment includes an opening/closing degree detection sensor 307, a notification unit 308, and the storage unit 309 in addition to the main body 302, the door 303, the transmitter 304, the receiver 305, and the controller 306 described above.

The opening/closing degree detection sensor 307 is configured to be capable of detecting the degree of opening/closing of the door 303 with respect to the main body 302. The controller 306 acquires opening/closing degree data of the door 303 from the opening/closing degree detection sensor 307. The controller 306 stores the opening/closing degree data of the door 303 acquired from the opening/closing degree detection sensor 307 and reception intensity data acquired from the receiver 305 in the storage unit 309 or the like in association with each other. The controller 306 compares variation data of the reception intensity according to the degree of opening/closing of the door 303 for the tube 203 accommodated in the accommodating groove 320 based on the opening/closing degree data and the reception intensity data acquired as described above with variation data of the reception intensity according to the degree of opening/closing of the door 303 for the liquid-filled tube 203a without external liquid adhesion stored in advance in the storage unit 309 or the like. As a result, the controller 306 determines the presence or absence of a void in the tube 203 accommodated in the accommodating groove 320, and determines whether the tube 203 accommodated in the accommodating groove 320 is the liquid-filled tube 203a without external liquid adhesion or the empty tube 203b with external liquid adhesion.

The opening/closing degree detection sensor 307 may be, for example, a Hall sensor including a Hall element capable of detecting a rotation angle of the door 303 with respect to the main body 302. However, the opening/closing degree detection sensor 307 is not limited to the Hall sensor. The opening/closing degree detection sensor 307 may be, for example, a potentiometer, a millimeter wave sensor, a laser sensor, or the like.

In addition, the tube void determination device 301 may further include an opening/closing sensor capable of detecting whether or not the door 303 is closed with respect to the main body 302. The controller 306 may execute the above-described comparison of the variation data of the reception intensity according to the degree of opening/closing of the door 303 based on the detection of the closed state by the opening/closing sensor.

Further, the tube void determination device 301 may have a configuration in which the opening/closing sensor is provided and the opening/closing degree detection sensor 307 is not provided. In such a case, at a time point when the closed state is detected by the opening/closing sensor, the controller 306 may compare pieces of the reception intensity data in a predetermined time before the time point. That is, the degree of opening/closing of the door 303 may be replaced with time, and whether the tube 203 accommodated in the accommodating groove 320 is the liquid-filled tube 203a without external liquid adhesion or the empty tube 203b with external liquid adhesion may be determined using a time history of the reception intensity data. In such a case, a time history of the reception intensity of the liquid-filled tube 203a without external liquid adhesion serving as a reference may be stored in advance in the storage unit 309. However, it is preferable to provide the opening/closing degree detection sensor 307 in order to accurately compare variations in reception intensities according to the degree of opening/closing of the door 303.

The notification unit 308 can notify the outside of a determination result in the controller 306. The notification unit 308 may be configured in any aspect capable of notifying the outside of the determination result. For example, the notification unit 308 may include a light emitting element and output the determination result to the outside according to a light emission color, a lighting state, a blinking state, or the like. In addition, the notification unit 308 may include, for example, a speaker and output the determination result to the outside by a sound such as an alarm sound or a voice announcement. In addition, the notification unit 308 may include, for example, a display device such as a liquid crystal display (LCD), an organic electro-luminescence display (OELD), or an inorganic electro-luminescence display (IELD) and output the determination result to the outside by various displays. In addition, the notification unit 308 may include, for example, a vibrator and output the determination result to the outside according to a vibration pattern. The notification unit 308 may be configured in an aspect other than the specific examples described herein. In addition, the notification unit 308 may be configured by combining a plurality of aspects.

The storage unit 309 may include a semiconductor memory, a magnetic memory, or the like. The storage unit 309 stores, for example, various kinds of data such as the reception intensity data acquired from the receiver 305, the opening/closing degree data acquired from the opening/closing degree detection sensor 307, the variation data of the reception intensity according to the degree of opening/closing of the door 303 for the liquid-filled tube 203a without external liquid adhesion, the program for operating the tube void determination device 301, and the like.

As illustrated in Figs. 1 and 2, in the tube void determination device 301 of the present embodiment, the transmitter 304, the receiver 305, the controller 306, the opening/closing degree detection sensor 307, the notification unit 308, and the storage unit 309 are provided in the main body 302, but the present invention is not limited to this configuration. The transmitter 304, the receiver 305, the controller 306, the opening/closing degree detection sensor 307, the notification unit 308, and the storage unit 309 may be provided in the door 303, for example. In addition, the transmitter 304, the receiver 305, the controller 306, the opening/closing degree detection sensor 307, the notification unit 308, and the storage unit 309 may be provided separately from the main body 302 and the door 303.

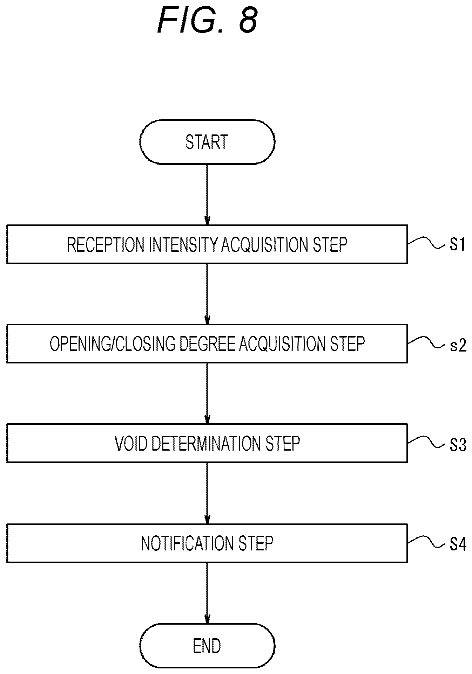

Fig. 8 is a view illustrating an example of a void determination method by the controller 306 according to the present embodiment. As illustrated in Fig. 8, the void determination method performed by the controller 306 according to the present embodiment includes a reception intensity acquisition step S1, an opening/closing degree acquisition step S2, a void determination step S3, and a notification step S4. Specifically, in the reception intensity acquisition step S1, the controller 306 according to the present embodiment acquires reception intensity data from the receiver 305. In the opening/closing degree acquisition step S2, the controller 306 acquires opening/closing degree data of the door 303 from the opening/closing degree detection sensor 307. The reception intensity acquisition step S1 and the opening/closing degree acquisition step S2 may be performed in the opposite order or at the same time.

Next, in the void determination step S3, the controller 306 of the present embodiment generates variation data of a reception intensity according to the degree of opening/closing of the door 303 for the tube 203 accommodated in the accommodating groove 320 based on the reception intensity data and the opening/closing degree data acquired in the reception intensity acquisition step S1 and the opening/closing degree acquisition step S2, respectively.

In addition, in the void determination step S3, the controller 306 of the present embodiment compares a maximum value of the reception intensity in the generated variation data with the predetermined first threshold. As a result, the controller 306 determines whether or not the tube 203 accommodated in the accommodating groove 320 is a liquid-filled tube with external liquid adhesion.

Further, in the void determination step S3, the controller 306 of the present embodiment compares the maximum value of the reception intensity in the generated variation data with the predetermined second threshold. As a result, the controller 306 determines whether or not the tube 203 accommodated in the accommodating groove 320 is an empty tube without external liquid adhesion.

When the controller 306 of the present embodiment determines that the tube 203 accommodated in the accommodating groove 320 is not the liquid-filled tube with external liquid adhesion and is not the empty tube without external liquid adhesion, the controller 306 compares the generated variation data with variation data of a reception intensity according to the degree of opening/closing of the door 303 for the liquid-filled tube 203a without external liquid adhesion stored in advance in the storage unit 309 or the like. As a result, the controller 306 determines whether the tube 203 accommodated in the accommodating groove 320 is the liquid-filled tube 203a without external liquid adhesion or the empty tube 203b with external liquid adhesion.

By performing the above series of determination in the void determination step S3, the controller 306 can determine the presence or absence of a void in the tube 203 accommodated in the accommodating groove 320 regardless of whether or not the liquid X2 (see Figs. 5 and 6) adheres to the outer surface. That is, the controller 306 can determine whether the tube is the liquid-filled tube or the empty tube regardless of whether or not the liquid X2 (see Figs. 5 and 6) adheres to the outer surface.

In addition, for example, the controller 306 may further consider an environmental temperature to determine the presence or absence of a void in the tube 203 accommodated in the accommodating groove 320. The hardness of the tube 203 may vary depending on the environmental temperature. Therefore, a deformation amount and a deformation posture of the tube 203 when deformed by being pressed by the door 303 can vary depending on the environmental temperature. That is, the reception intensity received by the receiver 305 can also vary depending on the environmental temperature. Therefore, the controller 306 may determine the presence or absence of a void in the tube 203 accommodated in the accommodating groove 320 based on the environmental temperature in addition to the reception intensity described above. The environmental temperature may be detected by, for example, a temperature detection sensor.

In the notification step S4, the controller 306 of the present embodiment notifies the outside of a determination result in the void determination step S3 using the notification unit 308. Specifically, when it is determined that there is a void in the tube 203, the controller 306 of the present embodiment causes the notification unit 308 to notify that the tube 203 is the empty tube. On the other hand, when it is determined that there is no void in the tube 203, the controller 306 of the present embodiment causes the notification unit 308 to notify that the tube 203 is the liquid-filled tube.

Here, the tubes 203 having different tube diameters and made of different tube materials may be accommodated in the accommodating groove 320. Further, an accommodating position of the tube 203 accommodated in the accommodating groove 320 is also likely to vary depending on a user. Further, there is an individual difference in the output of the transmitter 304 and the sensitivity of the receiver 305. These variations also affect the determination accuracy of the tube void determination device 301. That is, an output value output from the receiver 305 to the controller 306 varies depending on the presence or absence of a void in the tube 203 as described above, but also varies depending on various factors listed above, such as the tube diameter. If it can be determined that variations in the output value output from the receiver 305 to the controller 306 are caused by the various factors listed above, for example, the variations in the output value output from the receiver 305 to the controller 306 due to the difference in the tube diameter, the tube material, or the like of the tube 203 can be corrected by automatically adjusting the sensitivity of the receiver 305 or the like. As described above, by correcting the variations in the output value output from the receiver 305 to the controller 306, it is possible to suppress a decrease in the determination accuracy for a void in the tube 203 due to the difference in the tube diameter or the like. Conversely, in a case where it cannot be determined that the variations in the output value output from the receiver 305 to the controller 306 are caused by the various factors listed above, there is a possibility that the variations are caused by the presence or absence of a void in the tube 203, and thus, it is difficult to perform the automatic adjustment of the sensitivity of the receiver 305 or the like. Therefore, the tube void determination device 301 is used to determine in advance whether the tube 203 accommodated in the accommodating groove 320 is a liquid-filled tube or an empty tube, so that it is possible to perform the above automatic adjustment of the sensitivity of the receiver 305 or the like. Although the automatic adjustment of the sensitivity of the receiver 305 has been exemplified here, for example, automatic adjustment of the output of the transmitter 304 may be performed. In addition, both the automatic adjustment of the sensitivity of the receiver 305 and the automatic adjustment of the output of the transmitter 304 may be performed.

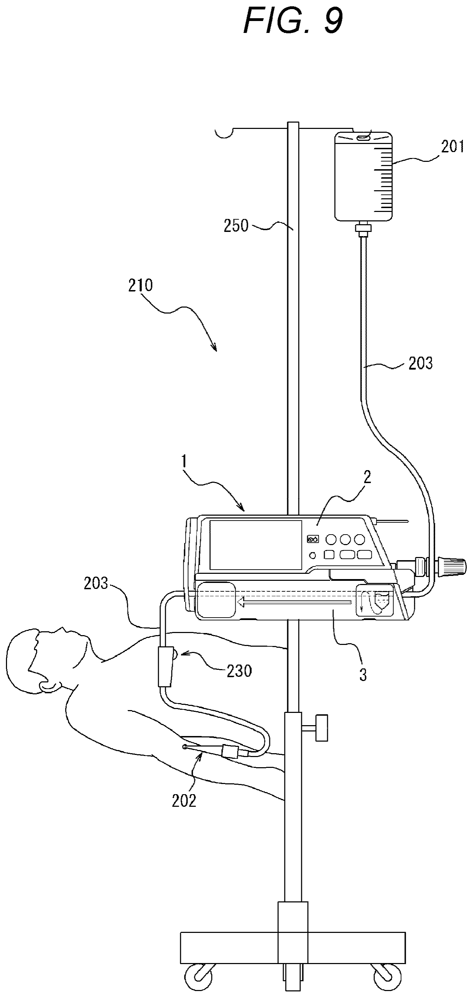

Next, a liquid delivery pump 1 as an embodiment of the liquid delivery pump according to the present disclosure including the above-described tube void determination device 301 will be described with reference to Figs. 9 to 12. Fig. 9 is a view illustrating a liquid delivery line 210 to which the liquid delivery pump 1 is attached. The liquid delivery pump 1 illustrated in Fig. 9 is an infusion pump, and the liquid delivery line 210 illustrated in Fig. 9 is an infusion line.

The liquid delivery line 210 illustrated in Fig. 9 is configured by connecting, with the tube 203 for an infusion, from an infusion container 201 that stores liquid such as a medicine to an indwelling needle 202 placed in the state of piercing a patient. Hereinafter, the infusion container 201 side of the liquid delivery line 210 may be referred to as a "flow path upstream side". In addition, the indwelling needle 202 side of the liquid delivery line 210 may be referred to as a "flow path downstream side". Further, hereinafter, a direction from the flow path upstream side to the flow path downstream side of the liquid delivery line 210 may be referred to as a liquid delivery direction A.

The infusion container 201 illustrated in Fig. 9 is suspended from a stand 250 and connected to the indwelling needle 202 via the tube 203. The liquid delivery pump 1 is attached to the tube 203 of the liquid delivery line 210 illustrated in Fig. 9. The liquid delivery pump 1 is fixed to a pole portion of the stand 250. Further, a clamp 230 is attached to a position of the tube 203 of the liquid delivery line 210 on the flow path downstream side of the liquid delivery pump 1 illustrated in Fig. 9.

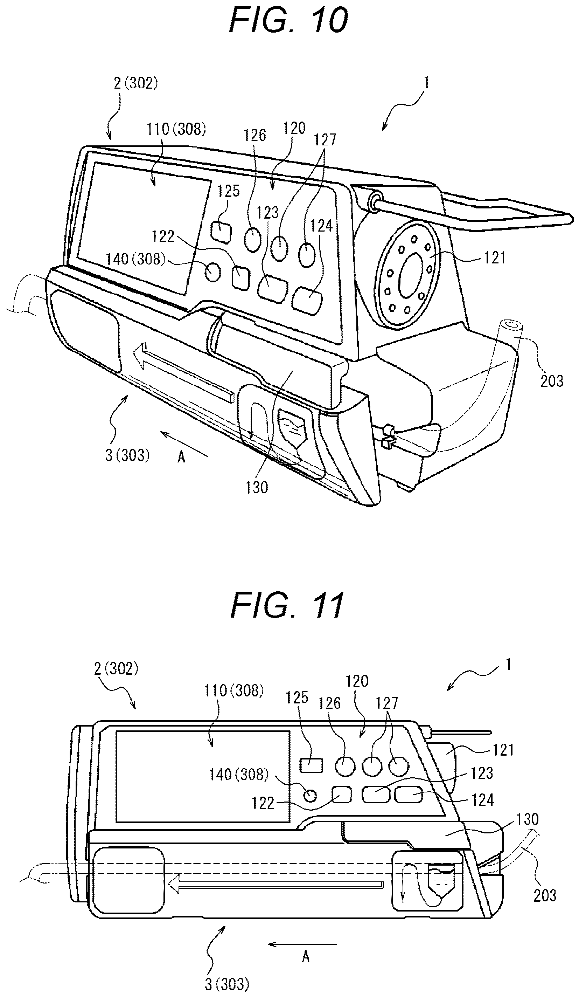

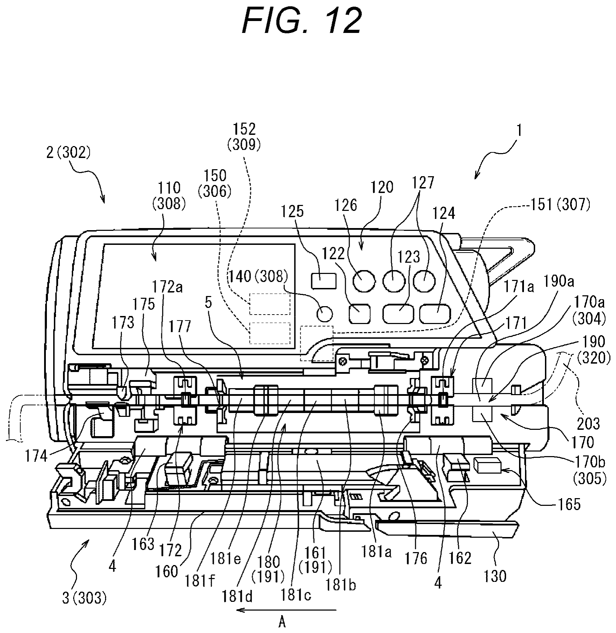

Fig. 10 is a front side perspective view of the liquid delivery pump 1 illustrated in Fig. 9. Figs. 11 and 12 are front views of the liquid delivery pump 1 illustrated in Fig. 9. In Figs. 10 to 12, the tube 203 attached to the liquid delivery pump 1 is indicated by a two-dot chain line.

As illustrated in Figs. 10 to 12, the liquid delivery pump 1 includes a main body 2 and a door 3 attached to the main body 2 so as to be openable and closable. Figs. 10 and 11 illustrate a state in which the door 3 is closed with respect to the main body 2. Fig. 12 illustrates a state in which the door 3 is opened with respect to the main body 2. As illustrated in Figs. 10 to 12, the liquid delivery pump 1 can hold the tube 203 at a position between the main body 2 and the door 3. The liquid such as a medicine stored in the infusion container 201 (see Fig. 9) is delivered in the liquid delivery direction A through the tube 203 by the liquid delivery pump 1, and injected into a blood vessel of a patient through the indwelling needle 202 (see Fig. 9).

As illustrated in Figs. 10 to 12, a display unit 110, an operation unit 120, a door lock lever 130, and an operation indicator 140 are provided in a front portion of the liquid delivery pump 1.

Various types of information are displayed on the display unit 110. Specifically, the display unit 110 of the present embodiment can display various types of information of the liquid delivery pump 1, such as a set value and an actual value of a flow rate (liquid delivery speed (mL/h)) per unit time, a scheduled amount and an integrated amount of a medicine to be administered to the patient, various alarms regarding operation states of the liquid delivery pump 1, a set level of occlusion detection sensitivity of the tube 203 by a downstream occlusion sensor 172 described later, the presence or absence of connection with an AC power supply, and a remaining capacity of a built-in battery. The display unit 110 may include, for example, an image display device such as a color liquid crystal display device. In addition, examples of the various alarms displayed on the display unit 110 include an air bubble sign displayed when an air bubble is detected in the tube 203 by an air bubble sensor 170 described later, a battery sign displayed when a voltage of the built-in battery of the liquid delivery pump 1 decreases, an occlusion sign displayed when an upstream occlusion sensor 171 or the downstream occlusion sensor 172 described later determines that the tube 203 is occluded, a door sign displayed when the door 3 is opened with respect to the main body 2, a completion sign displayed when an infusion is completed, and the like.

Operation switches and the like are arranged in the operation unit 120. Specifically, the operation unit 120 of the present embodiment includes a fast delivery switch 122 that enables liquid delivery at a liquid delivery speed higher than a set predetermined liquid delivery speed (mL/h) while being pressed, a start switch 123 that starts liquid delivery by being pressed, a stop switch 124 that stops liquid delivery by being pressed, a power switch 125 for operating ON/OFF of a power supply of the liquid delivery pump 1, a menu selection switch 126, and a mode switching switch 127 that switches to a mute mode by being pressed. In addition, as illustrated in Figs. 10 to 12, a setting dial 121 is provided on a side surface portion of the main body 2, and the liquid delivery speed, the scheduled amount, and the like can be changed by rotating the setting dial 121.

The door lock lever 130 is used in opening and closing operations of the door 3 with respect to the main body 2, and is configured to be lockable in a state in which the door 3 is closed. Specifically, the door lock lever 130 is used to open and close the door 3. The door lock lever 130 locks the door 3 with respect to the main body 2 such that the door 3 is not opened with respect to the main body 2 in the state in which the door 3 is closed with respect to the main body 2 (see Figs. 10 and 11). In addition, a locked state of the door 3 with respect to the main body 2 can be released by operating the door lock lever 130 from the state in which the door 3 is closed with respect to the main body 2 (see Figs. 10 and 11). When the locked state of the door 3 is released, it is possible to perform the operation of opening the door 3 with respect to the main body 2.

The operation indicator 140 is turned on or off, or blinks to indicate an operation state of the liquid delivery pump 1 to the outside according to the operation state. Specifically, a light emitting diode that emits red light and green light is built in the operation indicator 140 of the present embodiment. For example, the operation indicator 140 blinks in green when a liquid delivery operation at a normal speed or a fast liquid delivery operation is performed, is turned off in a stop state in which no alarm is issued and liquid delivery is not performed, blinks in red in a stop state caused by issuance of an alarm, and blinks alternately in green and red during a self-check or the like.

Next, a configuration of an inner surface of the door 3 of the liquid delivery pump 1 and a configuration of the main body 2 of the liquid delivery pump 1 will be described.

As illustrated in Fig. 12, the door 3 is rotatably attached to the main body 2 via a hinge 4. Thus, the door 3 is openable and closable with respect to the main body 2.

The inner surface of the door 3 is provided with a sealing member 160 that prevents the liquid such as a medicine from entering the main body 2 when the door 3 is closed, a tube pressing portion 161 that presses the tube 203 toward each of fingers 181a to 181f of the main body 2, an upstream pressing portion 162 that clamps the tube 203 with the upstream occlusion sensor 171 of the main body 2, a downstream pressing portion 163 that clamps the tube 203 with the downstream occlusion sensor 172 of the main body 2, and a pressing portion 165 that presses the tube 203 accommodated in an accommodating groove 190 of the air bubble sensor 170 of the main body 2 toward a groove bottom 190a of the accommodating groove 190 when the door 3 is closed with respect to the main body 2.

The sealing member 160 is formed using, for example, a resin material such as elastomer, and seals a portion between the main body 2 and the door 3 when the door 3 is closed with respect to the main body 2, thereby suppressing foreign matter such as the liquid from entering a position between the main body 2 and the door 3 from the outside.

When the tube 203 is pressed by the fingers 181a to 181f, the tube pressing portion 161 supports a back surface of the tube 203 opposite to a surface on the finger 181a to 181f side, and implements peristaltic movement of the tube 203 by pressing of the fingers 181a to 181f.

The main body 2 of the liquid delivery pump 1 is provided with the air bubble sensor 170 capable of detecting the presence or absence of an air bubble in the tube 203. The air bubble sensor 170 includes an ultrasonic transmitter 170a as the transmitter 304 and an ultrasonic receiver 170b as the receiver 305. The main body 2 of the liquid delivery pump 1 is provided with the upstream occlusion sensor 171 and the downstream occlusion sensor 172 that detect a change in internal pressure of the tube 203. The main body 2 of the liquid delivery pump 1 is provided with a clamp portion 173 that automatically clamps the tube 203 when the door 3 is opened to occlude a flow path in the tube 203, and a release lever 174 that releases the clamping of the tube 203 by the clamp portion 173. The main body 2 of the liquid delivery pump 1 is provided with an anti-free flow mechanism 175 including a clip that is detachably attached to the main body 2 and occludes the flow path in the tube 203 by clamping the tube 203 when the door 3 is opened. The main body 2 of the liquid delivery pump 1 is provided with a pump mechanism 180 that includes the fingers 181a to 181f and performs the liquid delivery operation in the liquid delivery direction A via the tube 203.

A tube attachment unit 5 to which the tube 203 can be attached is provided in a front portion of the main body 2 of the liquid delivery pump 1. In the tube attachment unit 5, the air bubble sensor 170, the upstream occlusion sensor 171, the fingers 181a to 181f, the downstream occlusion sensor 172, the anti-free flow mechanism 175, and the clamp portion 173 are arranged in this order from the flow path upstream side toward the flow path downstream side. In addition, in the tube attachment unit 5, a first tube guide portion 176 that is formed in a recessed shape, accommodates the tube 203, and assists the attachment of the tube 203 is provided between the upstream occlusion sensor 171 and the fingers 181a to 181f. Further, a second tube guide portion 177 that is formed in a recessed shape, accommodates the tube 203, and assists the attachment of the tube 203 is also provided between the fingers 181a to 181f and the downstream occlusion sensor 172 in the tube attachment unit 5. Further, the tube attachment unit 5 is provided with the accommodating groove 190 for holding the tube 203 between the ultrasonic transmitter 170a and the ultrasonic receiver 170b of the air bubble sensor 170.

The air bubble sensor 170 detects an air bubble mixed in the tube 203. When the air bubble is detected by the air bubble sensor 170, the air bubble sign is turned on in the display unit 110 illustrated in Fig. 10 to issue a notification for such a fact. The air bubble sensor 170 of the present embodiment can detect the air bubble in the tube 203 using an ultrasonic wave.

The upstream occlusion sensor 171 can measure the internal pressure of the tube 203 by detecting a movement of a plunger 171a illustrated in Fig. 12 in the radial direction of the tube 203.

The plunger 171a is arranged so as to be constantly in contact with the outer surface of the tube 203. When the internal pressure of the tube 203 varies and an outer diameter of the tube 203 changes, the plunger 171a moves according to the amount of the change. For example, when the internal pressure of the tube 203 increases and the tube 203 is deformed to swell, the plunger 171a is pushed and moves outward in the radial direction of the tube 203. Similarly, when the internal pressure of the tube 203 decreases and the tube 203 is deformed to contract, the plunger 171a moves inward in the radial direction of the tube 203. The internal pressure of a site of the tube 203 in contact with the plunger 171a is detected based on the amount of movement of the plunger 171a.

A configuration of the upstream occlusion sensor 171 is not particularly limited as long as the internal pressure of the tube 203 can be measured. In addition, the downstream occlusion sensor 172 also has the same configuration as the upstream occlusion sensor 171. Specifically, the downstream occlusion sensor 172 includes a plunger 172a having the same configuration as the plunger 171a.

When the door 3 is opened with respect to the main body 2, the clamp portion 173 automatically compresses and closes the tube 203 located in the tube attachment unit 5. That is, when the door 3 is opened with respect to the main body 2, the release lever 174 moves in conjunction with this opening operation of the door 3, whereby the tube 203 is compressed and closed. Conversely, when the door 3 is closed with respect to the main body 2, the clamp portion 173 automatically releases the compressed and closed state of the tube 203 located in the tube attachment unit 5. That is, when the door 3 is closed with respect to the main body 2, the release lever 174 moves in conjunction with this closing operation of the door 3, and the compressed and closed state of the tube 203 is released.

The clip of the anti-free flow mechanism 175 is detachably attached to the tube attachment unit 5, and in the state of being attached to the tube attachment unit 5, automatically compresses and closes the tube 203 located in the tube attachment unit 5 similarly the clamp portion 173 described above when the door 3 is opened with respect to the main body 2. Further, when the door 3 is closed with respect to the main body 2, the clip of the anti-free flow mechanism 175 automatically releases the compressed and closed state of the tube 203 located in the tube attachment unit 5.

The pump mechanism 180 includes the plurality of fingers 181a to 181f arranged along the extending direction (in the present embodiment, the liquid delivery direction A and the opposite direction thereof) of the tube 203 attached to the tube attachment unit 5 of the main body 2, a cam shaft including a plurality of eccentric cams that individually move the plurality of fingers 181a to 181f, respectively, forward and backward with respect to the tube 203, and a drive motor that rotationally drives the cam shaft.

The pump mechanism 180 includes six fingers of the first finger 181a, the second finger 181b, the third finger 181c, the fourth finger 181d, the fifth finger 181e, and the sixth finger 181f arranged in this order from the flow path upstream side to the flow path downstream side. In addition, the cam shaft is provided with six eccentric cams, that is, first eccentric cam to sixth eccentric cam arranged to face the fingers 181a to 181f, respectively.

In the liquid delivery pump 1 of the present embodiment, the liquid delivery unit 191 includes the pump mechanism 180 of the main body 2 described above and the tube pressing portion 161 of the door 3 described above. The liquid delivery unit 191 can deliver the liquid inside the tube 203 in the liquid delivery direction A. However, a configuration of the liquid delivery unit 191 is not limited to the configuration illustrated in the present embodiment.

The door 3 covers the tube attachment unit 5 in the state of being closed with respect to the main body 2. Then, the tube 203 is held between the main body 2 and the door 3 as illustrated in Figs. 10 and 11. In addition, the liquid delivery pump 1 of the present embodiment is attached to the stand 250 such that the tube 203 attached to the tube attachment unit 5 extends along the horizontal direction as illustrated in Fig. 9.

Specifically, a stand attachment unit that can be attached to the stand 250 is provided on a back surface portion of the main body 2 of the liquid delivery pump 1. In a state in which the stand attachment unit is attached to the stand 250, the tube 203 attached to the tube attachment unit 5 extends along the horizontal direction. The stand attachment unit of the main body 2 is configured using, for example, a pole clamp or the like, and is attached to be detachable from the other portion of the main body 2 in the present embodiment.

As described above, the liquid delivery pump 1 includes the above-described tube void determination device 301 (see Fig. 1 and the like). More specifically, the main body 2 of the liquid delivery pump 1 of the present embodiment also serves as the main body 302 (see Fig. 1 and the like) of the tube void determination device 301. The accommodating groove 190 in the main body 2 of the liquid delivery pump 1 of the present embodiment also serves as the accommodating groove 320 (see Fig. 1 and the like) of the main body 302 of the tube void determination device 301. The door 3 of the liquid delivery pump 1 of the present embodiment also serves as the door 303 (see Fig. 1 and the like) of the tube void determination device 301. The ultrasonic transmitter 170a of the liquid delivery pump 1 of the present embodiment also serves as the transmitter 304 (see Fig. 1 and the like) of the tube void determination device 301. The ultrasonic receiver 170b of the liquid delivery pump 1 of the present embodiment also serves as the receiver 305 (see Fig. 1 and the like) of the tube void determination device 301. A controller 150 (see Fig. 12) of the liquid delivery pump 1 of the present embodiment also serves as the controller 306 (see Fig. 1 and the like) of the tube void determination device 301. The display unit 110 and the operation indicator 140 of the liquid delivery pump 1 of the present embodiment also serve as the notification unit 308 (see Fig. 1 and the like) of the tube void determination device 301. Further, an opening/closing degree detection sensor 151 (see Fig. 12) and a storage unit 152 (see Fig. 12) of the liquid delivery pump 1 of the present embodiment also serve as the opening/closing degree detection sensor 307 (see Fig. 1 and the like) and the storage unit 309 (see Fig. 1 and the like) of the tube void determination device 301, respectively.

That is, the liquid delivery pump 1 includes the main body 2 including the accommodating groove 190 capable of accommodating the tube 203, and the door 3 that is attached to the main body 2 to be openable and closable and is capable of pressing the tube 203 accommodated in the accommodating groove 190 toward the groove bottom 190a (see Fig. 12) of the accommodating groove 190 in conjunction with a closing operation with respect to the main body 2. In the present embodiment, in a process of the closing operation of the door 3, the pressing portion 165 protruding from the inner surface of the door 3 presses the tube 203 in the accommodating groove 190 toward the groove bottom 190a.

The ultrasonic transmitter 170a can transmit an ultrasonic wave to the tube 203 accommodated in the accommodating groove 190. The ultrasonic receiver 170b can receive the ultrasonic wave transmitted from the ultrasonic transmitter 170a and transmitted through the tube 203.

Then, the controller 150 can determine the presence or absence of a void in the tube 203 based on a reception intensity as the reception intensity of the ultrasonic wave received by the ultrasonic receiver 170b in the process of the closing operation of the door 3 with respect to the main body 2.

Arrangement positions of the ultrasonic transmitter 170a and the ultrasonic receiver 170b of the present embodiment may be reversed.

As described above, the liquid delivery pump 1 of the present embodiment includes the tube void determination device 301 (see Fig. 1 and the like) described above. More specifically, in the liquid delivery pump 1 of the present embodiment, the above-described tube void determination device 301 (see Fig. 1 and the like) is achieved by using the ultrasonic transmitter 170a and the ultrasonic receiver 170b of the air bubble sensor 170.

The configurations of the accommodating groove 190, the ultrasonic transmitter 170a, and the ultrasonic receiver 170b, and the relative positional relationship therebetween in the liquid delivery pump 1 of the present embodiment are similar to the configurations of the accommodating groove 320 (see Figs. 1 to 6), the transmitter 304 (see Figs. 1 to 6), and the receiver 305 (see Figs. 1 to 6), and the relative positional relationship therebetween in the tube void determination device 301 (see Fig. 1 and the like) described above, and thus, description thereof is omitted here.

In addition, in the liquid delivery pump 1 of the present embodiment, each component in the liquid delivery pump 1 also serves as each component of the tube void determination device 301 (see Fig. 1 and the like) described above as described above, but the present invention is not limited to this configuration. The liquid delivery pump 1 may separately include, for example, each of the dedicated components for the tube void determination device 301 (see Fig. 1 and the like) described above. As an example, the liquid delivery pump 1 may separately include the controller 306 for the tube void determination device 301 (see Fig. 1 and the like) described above in addition to the controller 150 that executes control of each unit of the liquid delivery pump 1. The same applies to the transmitter 304, the receiver 305, and the like. However, when the tube void determination device 301 (see Fig. 1 and the like) is configured using the air bubble sensor 170 of the liquid delivery pump 1 as in the present embodiment, it is possible to suppress the configuration of the liquid delivery pump 1 from being complicated due to additional mounting of the tube void determination device 301 (see Fig. 1 and the like).

As described above, since the liquid delivery pump 1 of the present embodiment includes the tube void determination device 301 (see Fig. 1 and the like) described above, it is possible to determine whether a liquid-filled tube is attached or an empty tube is attached to the tube attachment unit 5.

The tube void determination device and the liquid delivery pump according to the present disclosure are not limited to the specific configurations illustrated in the above-described embodiment, and various modifications, changes, and combinations are possible without departing from the claims. As illustrated in Fig. 9, the liquid delivery pump 1 described above is configured to be attached to the stand 250 such that the tube 203 attached to the tube attachment unit 5 extends along the horizontal direction, but is not limited to this configuration. For example, the liquid delivery pump may be configured to be attached to the stand 250 such that the tube 203 attached to the tube attachment unit 5 extends along the vertical direction.

### Industrial Applicability

The present disclosure relates to a tube void determination device and a liquid delivery pump.

### Reference Signs List

1 Liquid delivery pump
2 Main body
3 Door
4 Hinge
5 Tube attachment unit
110 Display unit
120 Operation unit
121 Setting dial
122 Fast delivery switch
123 Start switch
124 Stop switch
125 Power switch
126 Menu selection switch
127 Mode switching switch
130 Door lock lever
140 Operation indicator
150 Controller
151 Opening/closing degree detection sensor
152 Storage unit
160 Sealing member
161 Tube pressing portion
162 Upstream pressing portion
163 Downstream pressing portion
165 Pressing portion (example of part of liquid delivery unit)
170 Air bubble sensor
170a Ultrasonic transmitter (example of transmitter)
170b Ultrasonic receiver (example of receiver)
171 Upstream occlusion sensor
171a Plunger
172 Downstream occlusion sensor
172a Plunger
173 Clamp portion
174 Release lever
175 Free flow mechanism
176 First tube guide portion
177 Second tube guide portion
180 Pump mechanism
181a to 181f First to sixth fingers
190 Accommodating groove
190a Groove bottom of accommodating groove
191 Liquid delivery unit
201 Infusion container
202 Indwelling needle
203 Tube
203a Liquid-filled tube without external liquid adhesion
203b Empty tube with external liquid adhesion
210 Liquid delivery line
230 Clamp
250 Stand
301 Tube void determination device
302 Main body
303 Door
304 Transmitter
305 Receiver
306 Controller
307 Opening/closing degree detection sensor
308 Notification unit
309 Storage unit
320 Accommodating groove
320a Groove bottom of accommodating groove
320b1, 320b2 Groove wall of accommodating groove
321 Hinge
A Liquid delivery direction
X1 Liquid in tube
X2 Liquid adhering to outer surface of tube

## Claims

1. A tube void determination device comprising:
a main body including an accommodating groove capable of accommodating a tube;
a door attached to the main body to be openable and closable, the door being capable of pressing the tube accommodated in the accommodating groove toward a groove bottom of the accommodating groove in conjunction with a closing operation with respect to the main body;
a transmitter capable of transmitting an electromagnetic wave or a sound wave to the tube accommodated in the accommodating groove, and a receiver capable of receiving the electromagnetic wave or the sound wave transmitted from the transmitter and transmitted through or reflected by the tube; and
a controller that determines presence or absence of a void in the tube based on a reception intensity received by the receiver in a process of the closing operation of the door with respect to the main body.

2. The tube void determination device according to claim 1, wherein the transmitter and the receiver are arranged to face each other in a radial direction of the tube while sandwiching the tube accommodated in the accommodating groove.

3. The tube void determination device according to claim 2, wherein
the main body is capable of clamping the tube between two opposing groove walls of the accommodating groove,
the transmitter is capable of transmitting the electromagnetic wave or the sound wave to the tube through one groove wall of the two groove walls, and
the receiver is capable of receiving the electromagnetic wave or the sound wave transmitted through or reflected by the tube through another groove wall of the two groove walls.

4. The tube void determination device according to any one of claims 1 to 3, further comprising an opening/closing degree detection sensor capable of detecting a degree of opening/closing of the door with respect to the main body,
wherein the controller determines the presence or absence of the void in the tube based on a variation and a maximum value of the reception intensity received by the receiver in the process of the closing operation of the door with respect to the main body, and the degree of opening/closing of the door with respect to the main body.

5. A liquid delivery pump comprising:
the tube void determination device according to any one of claims 1 to 3; and
a liquid delivery unit capable of delivering liquid inside the tube.
